# EUROPEAN PATENT APPLICATION

(11) **EP 3 693 418 A1**
(43) Date of publication of application: **12.08.2020**
(21) Application number: 20154355.0
(22) Date of filing: 29.01.2020
(51) Int. Cl.: C09D 7/20, C09D 9/00, C12P 7/04, C12P 7/62

(54) **A SOLVENT COMPOSITION**

(30) Priority: 11.02.2019 IT 201900001899; 22.01.2020 IT 202000001153
(71) Applicant: Liberty Chemicals S.r.l., 21010 Arsago Seprio - Varese (IT)
(72) Inventor: GIANI, Renato, I-21010 Arsago Seprio - VARESE (IT); MANENTI, Alberto, I-21010 Arsago Seprio - VARESE (IT)
(74) Representative: Long, Giorgio

(57) **Abstract**

The present invention relates to a solvent composition, in particular a solvent composition with components of natural, non-petrochemical origin.

In particular, the present invention relates to a solvent composition based on compounds of vegetable origin deriving from the fermentation of carbohydrates, wherein in particular said carbohydrates are selected from glucose, fructose, sucrose, starches, cellulose and mixtures thereof.

## Description

### Field of the invention

The present invention relates to a solvent composition, in particular a solvent composition with components of natural, non-petrochemical origin.

### Background art

Solvents are chemical products widely used in several fields, including the field of paints and varnishes, compositions for industrial and domestic cleaning, inks for printing and the extractive industry. In particular, in the paint and varnish field, solvents are used both as diluents and to remove stains and traces of dye from solid surfaces, from work tools, such as brushes or rollers or the like, or even from parts of the user's body.

In addition to a high solvent power, the solvent compositions should therefore also have low toxicity, in particular those used for domestic use. Most of the solvents currently available on the market, on the other hand, do not have such a low toxicity, which creates problems both for the health of the user and for the subsequent dispersion in the environment. In fact, it would be necessary for the solvents normally used in the aforementioned fields to be biodegradable.

### Summary of the invention

The present invention addresses the problem of providing a solvent composition with a solvent power which is comparable to or even higher than that of common solvents used as paint thinners, such as, for example, white spirit, but which has a significantly lower toxicity, which is largely from renewable sources and which is as biodegradable as possible.

Therefore, the present invention relates to a solvent composition comprising components of non-petrochemical natural origin, as outlined in the appended claims.

The text of the appended claims forms an integral part of the present description.

Further features and advantages of the invention will become apparent from the following description of preferred embodiments thereof, provided by way of a nonlimiting example.

### Detailed description of the invention

The present invention relates to a solvent composition based on compounds of vegetable origin, in particular produced by the fermentation of carbohydrates, such as, by way of example, glucose, fructose, sucrose, starches and cellulose.

In preferred embodiments, the solvent composition of the invention comprises:
- from 20% to 80% by weight, more preferably from 30% to 70% by weight, of a lactic acid ester;
- from 10% to 50% by weight, more preferably from 20% to 50% by weight of aliphatic alcohols;
- up to 8 % by weight of an aliphatic acid ester.

In preferred embodiments, the lactic acid ester is ethyl lactate.

In preferred embodiments, the aliphatic alcohol is ethanol, propanol, butanol, pentanol, hexanol, heptanol, octanol or mixtures thereof, more preferably ethanol, isobutanol, isoamyl alcohol or mixtures thereof.

In preferred embodiments, the aliphatic acid ester is isoamyl acetate, ethyl caprylate, ethyl caprate, ethyl acetate or mixtures thereof.

In particularly preferred embodiments, the solvent composition of the invention comprises:
- from 30% to 70% by weight of ethyl lactate;
- from 20% to 50% by weight of isobutanol;
- up to 5% by weight of ethyl acetate.

The solvent composition of the invention may be obtained by known processes of alcoholic, lactic or malolactic fermentation of vegetable substrates containing a carbohydrate content greater than 60% by weight in the dried material. Preferred vegetable substrates are selected from: cereals, buckwheat, beet and sugar cane, tubers, fruit and mixtures thereof. For example, the processes described in WO 2018/192952 by Galactic SA or in WO 2005/108533 by Cargill Inc. may be used.

Cereals are preferably selected from wheat, corn, rice, barley, sorghum, millet, oats, rye, fonio, teff, hulled wheat, sorghum, spelt and mixtures thereof.

The tubers are preferably selected from potatoes, sweet potatoes, Jerusalem artichokes and mixtures thereof.

The solvent composition according to the invention is characterized by a low toxicity, a high carbon content from renewable sources and a total or almost total biodegradability.

In preferred embodiments, the solvent composition of the invention has a carbon content from renewable sources (i.e. of vegetable origin) greater than 65% by weight, more preferably greater than 66% by weight, even more preferably between 66.5% and 67% by weight. The carbon content from renewable sources was determined according to the method provided for by the European standard UNI EN 16640:2017, according to the AMS (Accelerator Mass Spectrometry) method.

The AMS method allows the direct determination of the content of ¹⁴C in the sample on the basis of a reference standard, from which the ¹⁴C/¹²C ratio of the analyzed sample can be calculated and from here, on the basis of known samples and by means of a curve calibration, the percentage of carbon from renewable sources is derived, with an error between 2% and 5%.

In preferred embodiments, the solvent composition of the invention has a boiling point in the range of 110-151 °C, a specific gravity at 25 °C of about 0.94 g/cm³ and a viscosity at 20 °C of 1.50 cPs (measured with Brookfield viscometer).

The solvent power of the composition of the invention containing ethyl lactate, isobutanol and ethyl acetate was tested in the percentage ratios defined above (sample A) in comparison with commercial solvent compositions. The results of the experiments are reported below.

### Aggression tests on painted panels

Measurement of the difference in "gloss" after 30 rubs according to the ASTM D 5264 standard with different solvents:

| **Paint type** | **Initial opacity** | **Treatment with white spirits** | **Treatment with Hammarite (Akzo Nobel)** | **treatment with sample A** |
|---|---|---|---|---|
| Transparent matte WB | 43 | 48 | 49 | 51 detectable aggression |
| UV matte | 6 | 8 | 8 | 8 |
| Matte white Nitro | 12 | Aggression and discoloration | Aggression and discoloration | High aggression and discoloration |

### Stain resistance assessment

The test is conducted according to the UNI EN 12720:2013 standard. The painted panel is kept in contact with the solvent for 6 hours, then signs of deterioration and damage to the panel surface are assessed. Lower values indicate greater aggressiveness of the solvent.

| **Paint type** | **White spirits** | **Sample A** |
|---|---|---|
| Matte white WB | 3 | 2 |
| Transparent matte WB | 4 | 3 |
| UV matte | 5 | 5 |
| Matte white PU | 4 | 4 |
| Matte transparent PU | 5 | 4 |
| Matte white Nitro | 2 | 1 |

The above data show a greater or equal aggressiveness of the composition according to the invention in comparison with the reference solvent compositions on the market.

### Evaluation of the cleaning power of the solvent composition

The cleaning power was determined by immersing a brush, previously wet in a paint and subsequently used to brush the surface of a wooden panel (see previous test), in a glass containing 250 ml of solvent composition and keeping it in immersion for a time of 120 seconds. After this time, the transmittance of the solution/dispersion of paint extracted by the brush due to the action of the solvent composition was measured.
Instrument: Zetalab UV-20 spectrophotometer
Wavelength: 500 nm
Optical path: 10 mm.

### Solvent composition Transmittance

Sample A 29.5%
Hammarite 50.2%
White spirits 58%.

These values indicate that the solvent composition of the invention has a significantly greater cleaning power than the two reference solvents on the market.

### EXAMPLE OF PREPARATION OF THE SOLVENT COMPOSITION OF THE INVENTION

In a mixer adapted to use and prepare flammable solvents, therefore in accordance with Atex, the three ingredients are added in the order, namely ethyl lactate, isobutanol, and finally ethyl acetate in a weight ratio of 70/25/5.

Mixing is carried out at room temperature for 20-30 minutes.

A representative sample is then taken for quality control assays. If the measured data are in accordance with the regulations, the finished product is unloaded in the appropriate packaging.

It is apparent that only some particular embodiments of the present invention have been described, and those skilled in the art will be able to make all the necessary modifications for its adaptation to particular applications, without departing from the protection scope of the present invention.

## Claims

1. A solvent composition based on compounds of vegetable origin deriving from the fermentation of carbohydrates.

2. The solvent composition according to claim 1, wherein said carbohydrates are selected from glucose, fructose, sucrose, starches, cellulose and mixtures thereof.

3. The solvent composition according to claim 1 or 2, comprising:
- from 20% to 80% by weight of a lactic acid ester;
- from 10% to 50% by weight of aliphatic alcohols or mixtures thereof;
- up to 8% by weight of an aliphatic acid ester.

4. The solvent composition according to any one of claims 1 to 3, comprising:
- from 30% to 70% by weight of a lactic acid ester;
- from 20% to 50% by weight of aliphatic alcohols or mixtures thereof;
- up to 8% by weight of an aliphatic acid ester.

5. The solvent composition according to any one of claims 3 to 4, wherein the aliphatic alcohol is selected from ethanol, propanol, butanol, pentanol, hexanol, heptanol, octanol or mixtures thereof, more preferably ethanol, isobutanol, isoamyl alcohol and mixtures thereof.

6. The solvent composition according to any one of claims 3 to 5, wherein the aliphatic acid ester is selected from isoamyl acetate, ethyl caprylate, ethyl caprate, ethyl acetate and mixtures thereof.

7. The solvent composition according to any of the preceding claims, said solvent composition comprising:
- from 30% to 70% by weight of ethyl lactate;
- from 20% to 50% by weight of isobutanol;
- up to 5% by weight of ethyl acetate.

8. The solvent composition according to any one of the preceding claims, having a carbon content from renewable sources of vegetable origin greater than 65% by weight, preferably greater than 66% by weight, more preferably between 66.5% and 67% by weight, as determined according to the AMS method provided by the European standard UNI EN 16640:2017.

9. The method of preparing the solvent composition according to any one of claims 1 to 8, comprising a step of alcoholic, lactic or malolactic fermentation of vegetable substrates containing a carbohydrate content higher than 60% by weight in the dried material, said vegetable substrates being preferably selected from cereals, buckwheat, beet and sugar cane, tubers, fruit and mixtures thereof, wherein the cereals are preferably selected from wheat, corn, rice, barley, sorghum, millet, oats, rye, fonio millet, teff, spelt, sorghum, and mixtures thereof; the tubers are preferably selected from potatoes, sweet potatoes, Jerusalem artichokes and mixtures thereof.
